# EUROPEAN PATENT APPLICATION

(11) **EP 4 832 145 A1**
(43) Date of publication of application: **28.10.2026**
(21) Application number: 26167998.9
(22) Date of filing: 26.03.2026
(51) Int. Cl.: H01M 10/52, H01M 10/42, H01M 10/48

(54) **POWER STORAGE DEVICE AND METHOD FOR DETECTING SULFIDE GAS**

(30) Priority: 25.04.2025 JP 2025073319
(71) Applicant: Toyota Jidosha Kabushiki Kaisha, Toyota-shi, Aichi-ken 471-8571 (JP)
(72) Inventor: SASAKI, Manabu, Toyota-shi, Aichi-ken, 471-8571 (JP)
(74) Representative: Winter, Brandl - Partnerschaft mbB

(57) **Abstract**

A battery pack (100) includes: a battery module (10) including a unit cell (11) having a solid electrolyte layer (3); a battery case (20) that accommodates the battery module (10); an absorber (60) that is disposed inside the battery case (20) and absorbs sulfide gas; a sensor unit (70) that measures an electrical characteristic of the absorber (60); and an arithmetic unit (50) that detects the sulfide gas absorbed by the absorber (60), based on the electrical characteristic measured by the sensor unit (70).

## Description

### CROSS REFERENCE TO RELATED APPLICATIONS

This nonprovisional application is based on Japanese Patent Application No. 2025-073319 filed on April 25, 2025 with the Japan Patent Office, the entire contents of which are hereby incorporated by reference.

### BACKGROUND

### Field

The present disclosure relates to a power storage device, and a method for detecting sulfide gas.

### Description of the Background Art

Japanese Patent Laying-Open No. 2025-19541 discloses a battery pack that determines generation of gas based on a voltage of a wire corroded by the gas generated from a unit cell.

### SUMMARY

In the battery pack described in Japanese Patent Laying-Open No. 2025-19541, it is conceivable that corrosion of the wire does not proceed when the amount of generated gas is small. In this case, it may not be possible to determine that the gas is generated.

The present disclosure has been made to solve the aforementioned problem, and an object thereof is to provide a power storage device and a method for detecting sulfide gas that are capable of easily determining generation of the sulfide gas.

A power storage device according to a first aspect of the present disclosure includes: a power storage module including a power storage cell having a sulfide solid electrolyte; a case that accommodates the power storage module; an absorber that is disposed inside the case and absorbs sulfide gas; a measurement unit that measures an electrical characteristic of the absorber; and a controller that detects the sulfide gas absorbed by the absorber, based on the electrical characteristic measured by the measurement unit.

In the power storage device according to the first aspect of the present disclosure, as described above, the sulfide gas is detected based on the electrical characteristic of the absorber that absorbs the sulfide gas. Here, it is relatively easy to cause the absorber capable of absorbing the sulfide gas to absorb the sulfide gas, than to cause a wire or the like to be corroded by the sulfide gas, for example. Therefore, generation of the sulfide gas can be easily determined based on the electrical characteristic of the absorber, when compared with a case where the sulfide gas is detected based on corrosion of the wire or the like.

The absorber may have water absorbency. With such a configuration, a sulfide aqueous solution formed by dissolving the sulfide gas in dew condensation water generated inside the case can be absorbed by the absorber.

The case may include a bottom plate in which a recess is formed. The absorber may be disposed in the recess. Here, since the sulfide gas is heavier than air, the sulfide gas is likely to move to a bottom plate side inside the case. Further, the sulfide aqueous solution formed by dissolving the sulfide gas in the dew condensation water moves to the bottom plate side by its own weight inside the case. Therefore, by disposing the absorber in the recess formed in the bottom plate, more sulfide gas can be absorbed by the absorber.

The absorber may be located below the power storage module. With such a configuration, the sulfide gas that moves below the power storage module can be easily absorbed by the absorber.

The absorber may be formed in a gel state. Thereby, unlike a case where the absorber is a liquid, it is possible to suppress the absorber from spattering due to vibration or the like. Since it is thereby possible to suppress the absorber that has absorbed the sulfide gas from spattering inside the case, it is possible to suppress corrosion inside the case.

The case may have a sealed structure. With such a configuration, it is possible to suppress water from entering the inside of the case from the outside of the case. As a result, it is possible to suppress the water entering from the outside of the case from being absorbed by the absorber. As a result, it is possible to suppress detection of the sulfide gas from becoming inappropriate due to the water entering from the outside of the case.

The power storage device may further include a gas detection unit that detects the sulfide gas inside the case. With such a configuration, in addition to detection of the sulfide gas by the controller, the sulfide gas can also be detected by the gas detection unit. Thereby, when an abnormality occurs in the detection of the sulfide gas by the controller, for example, generation of the sulfide gas can be determined based on a detection result of the gas detection unit.

The absorber has an upper end portion, a lower end portion, and a central portion between the upper end portion and the lower end portion, and the measurement unit is disposed in a portion of the absorber on a side closer to the upper end portion with respect to the central portion. With such a configuration, it is possible to measure an electrical characteristic of the portion of the absorber on the side closer to the upper end portion with respect to the center portion, by the measurement unit.

The measurement unit may include a substrate disposed inside the case. The absorber may cover a void between specific two points on the substrate. The measurement unit may measure a potential difference in the void between the two points covered with the absorber. The controller may calculate a resistance value in the void between the two points based on the potential difference, and detect the sulfide gas absorbed by the absorber based on the resistance value. With such a configuration, generation of the sulfide gas can be easily determined based on the resistance value in the void between the two points.

In this case, when the resistance value is less than a predetermined threshold value, the controller may determine that the absorber is in a first state, and when the resistance value is more than or equal to the predetermined threshold value, the controller may determine that the absorber is in a second state different from the first state. With such a configuration, it is possible to separately distinguish the state of the absorber into the first state and the second state, by using the predetermined threshold value.

The first state may include a state in which the absorber absorbs moisture and the sulfide gas, and the second state may include a state in which the absorber absorbs moisture without absorbing the sulfide gas. With such a configuration, it is possible to easily separately distinguish whether or not the sulfide gas is contained in the absorber.

A method for detecting sulfide gas according to a second aspect of the present disclosure is a method for detecting sulfide gas performed by a power storage device including a power storage module including a power storage cell having a sulfide solid electrolyte, the method including: measuring an electrical characteristic of an absorber that is disposed inside a case accommodating the power storage module and absorbs the sulfide gas; and detecting the sulfide gas absorbed by the absorber, based on the electrical characteristic.

In the method for detecting the sulfide gas according to the second aspect of the present disclosure, as described above, the sulfide gas is detected based on the electrical characteristic of the absorber that absorbs the sulfide gas. Thereby, it is possible to provide a method for detecting sulfide gas capable of easily determining generation of the sulfide gas based on an electrical characteristic of an absorber.

The foregoing and other objects, features, aspects and advantages of the present disclosure will become more apparent from the following detailed description of the present disclosure when taken in conjunction with the accompanying drawings.

### BRIEF DESCRIPTION OF THE DRAWINGS

Fig. 1 is a view showing configurations of a vehicle and a battery pack according to the present embodiment.
Fig. 2 is a cross sectional view taken along a line II-II in Fig. 1.
Fig. 3 is a top view showing a configuration of a unit cell.
Fig. 4 is a cross sectional view taken along a line IV-IV in Fig. 3.
Fig. 5 is a schematic perspective view showing a configuration inside a battery case.
Fig. 6 is a cross sectional view taken along a line VI-VI in Fig. 5.
Fig. 7 is a flowchart showing a method for detecting hydrogen sulfide performed by the battery pack.
Fig. 8 is a cross sectional view showing a configuration of an absorber according to a first modification of the present embodiment.
Fig. 9 is a cross sectional view showing a configuration of an absorber according to a second modification of the present embodiment.

### DESCRIPTION OF THE PREFERRED EMBODIMENTS

An embodiment of the present disclosure will be described with reference to the drawings. It should be noted that, in the drawings referred to below, the same or corresponding members will be designated by the same reference numerals.

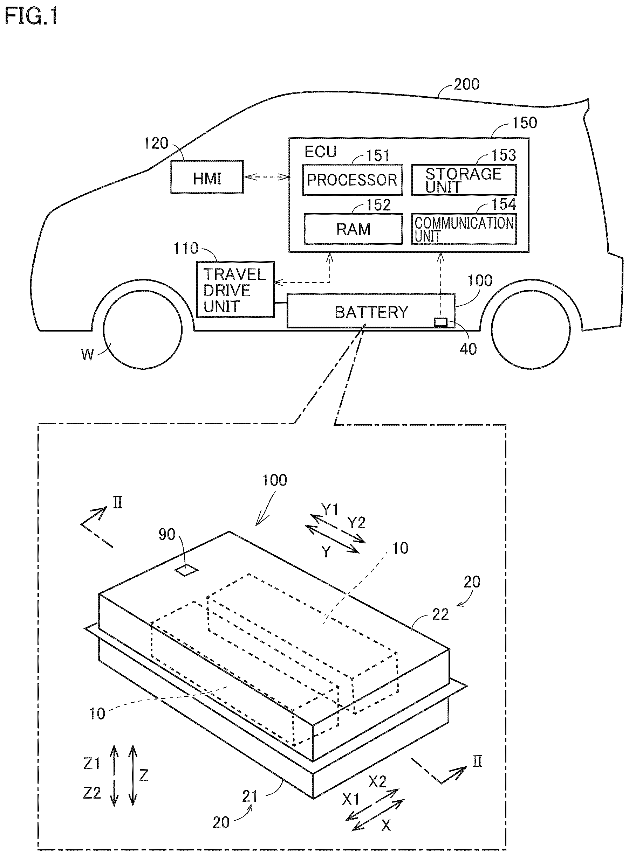

Fig. 1 is a view schematically showing an overall configuration of a vehicle 200 having a battery pack 100 mounted thereon. It should be noted that an X direction, a Y direction, and a Z direction shown in Fig. 1 are directions orthogonal to each other. For example, the X direction and the Y direction may be a vehicle width direction and a front-rear direction of vehicle 200, respectively, when battery pack 100 is mounted on vehicle 200. An X1 direction and an X2 direction may be a vehicle left direction and a vehicle right direction, respectively. A Y1 direction and a Y2 direction may be a vehicle front direction and a vehicle rear direction, respectively. Further, the Z direction may be an up-down (a vertical) direction. It should be noted that battery pack 100 is an example of the "power storage device" in the present disclosure.

Vehicle 200 includes battery pack 100 that stores power for traveling. Vehicle 200 is configured to travel using the power stored in battery pack 100. While the present embodiment describes a case where vehicle 200 is a battery electric vehicle (BEV) that does not include an engine (internal combustion engine) as an example, vehicle 200 may be a hybrid electric vehicle (HEV) or a plug-in hybrid electric vehicle (PHEV) that includes an engine. It should be noted that the application of battery pack 100 is not limited to that for a vehicle.

Vehicle 200 includes a control device (an electronic control unit: ECU) 150. ECU 150 is configured to perform charging control and discharging control for battery pack 100. ECU 150 is configured to include a processor 151, a random access memory (RAM) 152, a storage unit 153, and a communication unit 154. RAM 152 functions as a working memory that temporarily stores data to be processed by processor 151. Storage unit 153 stores a program, and information (for example, maps, mathematical expressions, and various parameters) to be used for the program. When processor 151 executes the program stored in storage unit 153, various types of control (charging control and discharging control for battery pack 100, and communication control using communication unit 154) in ECU 150 are performed. Communication unit 154 is configured to perform wireless communication with an external terminal (not shown) using a predetermined communication scheme.

It should be noted that battery pack 100 is charged by regenerative power from a travel drive unit 110 described later, or is charged (externally charged) by power supplied from a charging facility external to vehicle 200.

Vehicle 200 further includes travel drive unit 110, a human machine interface (HMI) device 120, and drive wheels W. Travel drive unit 110 includes a power control unit (PCU) and a motor generator (MG) (which are not shown), and is configured to drive the MG using the power stored in battery pack 100 to cause vehicle 200 to travel. Further, the MG is configured to perform regenerative power generation and supply generated power to battery pack 100.

HMI device 120 includes an input device and a display device. HMI device 120 may include a touch panel display.

Battery pack 100 includes a plurality of (two in the present embodiment) battery modules 10, and a battery case 20. Each of the plurality of battery modules 10 is accommodated in battery case 20. The plurality of battery modules 10 are disposed to be aligned in the X direction. Battery case 20 is made of metal (for example, aluminum). It should be noted that battery case 20 is an example of the "case" in the present disclosure. Further, battery module 10 is an example of the "power storage module" in the present disclosure.

Battery case 20 is constituted by a lower case 21 and an upper case 22. In the present embodiment, two battery modules 10 are accommodated in a space formed by lower case 21 and upper case 22. Battery pack 100 is mounted on a floor of vehicle 200. It should be noted that battery pack 100 may be mounted inside a vehicle compartment of vehicle 200, or may be mounted outside the vehicle compartment of vehicle 200.

Battery case 20 has a sealed structure. Battery pack 100 includes a breathing valve 90. Breathing valve 90 is provided to battery case 20. Breathing valve 90 is provided to upper case 22, for example. Breathing valve 90 adjusts a pressure inside battery case 20 by allowing gas (air) to pass therethrough. For example, breathing valve 90 adjusts the pressure inside battery case 20 when an internal pressure of battery case 20 decreases due to generation of dew condensation water inside battery case 20. It should be noted that the position where breathing valve 90 is disposed is not limited to the above example.

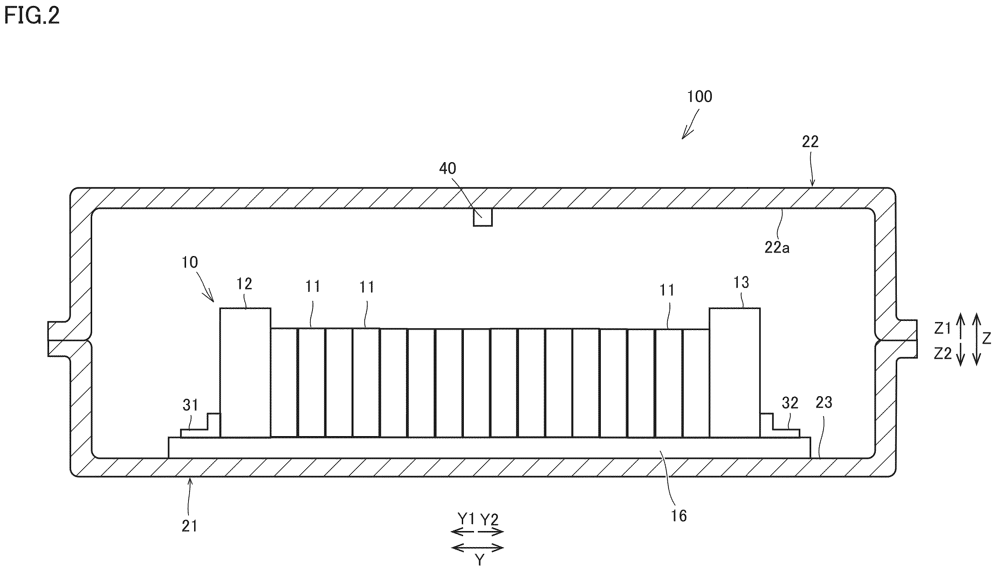

Fig. 2 is a view showing a schematic configuration of battery pack 100. Fig. 2 shows a cross section taken along **II-II** in Fig. 1. Battery module 10 is a battery assembly in which a plurality of unit cells 11 are connected. The plurality of unit cells 11 are stacked in the Y direction between a pair of end plates 12 and 13. The plurality of unit cells 11 in a stacked state are sandwiched between the pair of end plates 12 and 13, and a predetermined restraint load is applied thereto by a restraint band or the like (not shown). It should be noted that unit cell 11 is an example of the "power storage cell" in the present disclosure.

The pair of end plates 12 and 13 are fixed to a bottom plate 16 by brackets 31 and 32. Battery module 10 including unit cells 11 stacked between the pair of end plates 12 and 13 and bottom plate 16 is fixed to a bottom plate 23 of lower case 21. Battery case 20 is a housing that accommodates battery module 10.

A hydrogen sulfide sensor 40 is disposed inside battery case 20. Hydrogen sulfide sensor 40 is a sensor that detects a concentration C of hydrogen sulfide (H₂S) contained in an atmosphere (air inside battery pack 100), and outputs a voltage signal indicating a detection result to ECU 150 (Fig. 1). It should be noted that hydrogen sulfide sensor 40 is an example of the "gas detection unit" in the present disclosure. Further, hydrogen sulfide is an example of the "sulfide gas" in the present disclosure.

In the present embodiment, hydrogen sulfide sensor 40 is provided on a top plate 22a of upper case 22 or in the vicinity of top plate 22a. Thereby, hydrogen sulfide released from unit cell 11 toward a Z1 side can be easily sensed by hydrogen sulfide sensor 40. It should be noted that the position where hydrogen sulfide sensor 40 is disposed is not limited to the above example. For example, hydrogen sulfide sensor 40 may be disposed on bottom plate 23 of lower case 21.

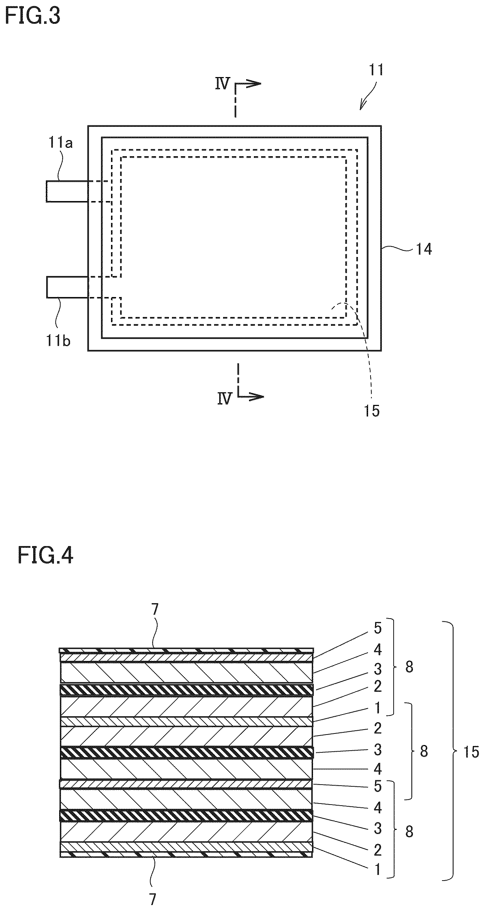

Fig. 3 is a top view for illustrating a schematic configuration of unit cell 11 according to the present embodiment. Unit cell 11 is a laminated all-solid-state battery using a laminate film as an exterior member 14, and a negative electrode terminal (negative electrode tab) 11a and a positive electrode terminal (positive electrode tab) 11b protrude from exterior member 14. The laminate film may be, for example, a pouch made of an aluminum laminate film, or may be a film having a three-layer structure including an aluminum foil sandwiched between resin films. It should be noted that, as exterior member 14, a metal can may be used instead of the laminate film.

Fig. 4 shows an all-solid-state battery stack 15 accommodated in exterior member 14, and shows a cross section taken along IV-IV in Fig. 3. All-solid-state battery stack 15 includes three all-solid-state battery elements 8, each of which is constituted by stacking a negative electrode current collector layer 1, a negative electrode active material layer 2, a solid electrolyte layer 3, a positive electrode active material layer 4, and a positive electrode current collector layer 5 in this order, and three all-solid-state battery elements 8 are stacked in such a manner that the order in which the layers are stacked is opposite with respect to each other, so as to share negative electrode current collector layer 1 and positive electrode current collector layer 5. Negative electrode current collector layer 1 is connected to negative electrode terminal 11a, and positive electrode current collector layer 5 is connected to positive electrode terminal 11b. It should be noted that the number of all-solid-state battery elements 8 included in all-solid-state battery stack 15 may be one, or may be four or more. An insulating film 7 insulates all-solid-state battery stack 15 from exterior member 14 (laminate film). It should be noted that, instead of a stacked type as described above, unit cell 11 may be of a wound type in which a negative electrode current collector layer, a negative electrode active material layer, a solid electrolyte layer, a positive electrode active material layer, and a positive electrode current collector layer are stacked and wound. It should be noted that solid electrolyte layer 3 is an example of the "sulfide solid electrolyte" in the present disclosure.

Unit cell 11 is a sulfide-based all-solid-state battery. In the present disclosure, the sulfide-based all-solid-state battery is a battery in which at least one of a material for negative electrode active material layer 2, a material for positive electrode active material layer 4, and a material for solid electrolyte layer 3 contains a sulfur component. In the present embodiment, solid electrolyte layer 3 contains a sulfide-based solid electrolyte, and for example, the sulfide-based solid electrolyte may be obtained from phosphorus tetrasulfide (P₂S₅) or lithium sulfide (Li₂S) as a starting material. In this case, positive electrode active material layer 4 may contain lithium cobaltate, lithium nickelate, lithium iron phosphate, or the like, for example. When solid electrolyte layer 3 is composed of an oxide-based solid electrolyte, a sulfur-based positive electrode active material may be used for positive electrode active material layer 4. The sulfur-based positive electrode active material may be an organic sulfur compound or an inorganic sulfur compound. It should be noted that two or all of negative electrode active material layer 2, positive electrode active material layer 4, and solid electrolyte layer 3 may contain a sulfur component (sulfide-based solid electrolyte).

Air may enter unit cell 11 from, for example, a sealed portion of exterior member 14 (laminate film). If the entering air contains moisture, the sulfur component contained in solid electrolyte layer 3, negative electrode active material layer 2, or positive electrode active material layer 4 may react with the moisture to generate hydrogen sulfide, which may be released into battery case 20.

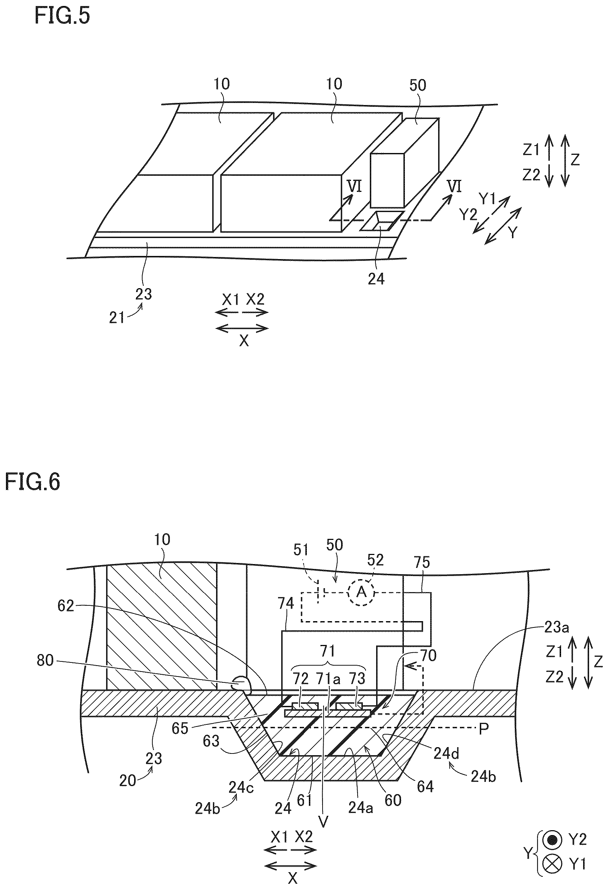

Fig. 5 is a schematic perspective view showing a configuration inside battery case 20. In Fig. 5, illustration of wires 74 and 75 and an absorber 60, which will be described later, is omitted for simplicity.

Battery pack 100 includes an arithmetic unit 50. Arithmetic unit 50 is disposed inside battery case 20. Arithmetic unit 50 is a monitoring IC that monitors a voltage of each battery module 10 or the like. It should be noted that arithmetic unit 50 may be disposed outside battery case 20. Further, arithmetic unit 50 is an example of the "controller" in the present disclosure.

A recess 24 is formed in bottom plate 23 of lower case 21. Recess 24 is disposed in the vicinity of arithmetic unit 50. In the example shown in Fig. 5, recess 24 is formed at a position adjacent to arithmetic unit 50 on a Y2 side of arithmetic unit 50.

Fig. 6 shows a cross section of recess 24 taken along the X direction, and shows a cross section taken along VI-VI in Fig. 5.

Battery pack 100 includes absorber 60 and a sensor unit 70. Absorber 60 and sensor unit 70 are disposed inside battery case 20. It should be noted that sensor unit 70 is an example of the "measurement unit" in the present disclosure.

Absorber 60 absorbs hydrogen sulfide. Specifically, absorber 60 absorbs hydrogen sulfide water 80 formed by dissolving hydrogen sulfide (gas) in the dew condensation water inside battery case 20 (i.e., absorbs moisture). It should be noted that absorber 60 may be capable of directly absorbing hydrogen sulfide (gas) inside battery case 20. Further, hydrogen sulfide water 80 is an example of the "sulfide aqueous solution" in the present disclosure.

Absorber 60 may be made of glycerin or the like, for example. Glycerin is a polymer resin having a high acid resistance. Thereby, it is possible to suppress absorber 60 from being deteriorated by absorbing the sulfide aqueous solution.

Sensor unit 70 measures an electrical characteristic of absorber 60. Specifically, sensor unit 70 measures a potential difference in a void between two points in absorber 60. Sensor unit 70 is disposed inside absorber 60. A method for measuring the potential difference with sensor unit 70 will be described later.

Here, in a conventional battery pack that detects generation of gas from a unit cell based on the degree of corrosion of a wire or the like, for example, corrosion of the wire does not proceed when the amount of generated gas is small, and thus it may not be possible to determine that the gas is generated.

Accordingly, in the present embodiment, arithmetic unit 50 detects hydrogen sulfide (hydrogen sulfide water) absorbed by absorber 60, based on the electrical characteristic of absorber 60 measured by sensor unit 70. Specifically, arithmetic unit 50 detects hydrogen sulfide absorbed by absorber 60, by detecting a decrease in a resistance value of absorber 60 due to absorption of hydrogen sulfide. Arithmetic unit 50 may be capable of calculating a concentration of hydrogen sulfide with respect to moisture absorbed by absorber 60.

Thereby, unlike a case where generation of gas from a unit cell is detected based on the degree of corrosion of a wire or the like, for example, hydrogen sulfide can be detected easily and quickly without requiring time to wait for a wire to be corroded or the like.

Absorber 60 is formed in a gel state (a state in which a colloidal solution is solidified like jelly). That is, absorber 60 has viscosity. Further, absorber 60 has water absorbency and moisture retention.

Recess 24 is defined by a bottom surface 24a and a peripheral surface 24b. Peripheral surface 24b extends toward the Z1 side from an outer peripheral edge of bottom surface 24a. Peripheral surface 24b extends downward from a bottom surface 23a of lower case 21 on which battery module 10 and arithmetic unit 50 are disposed. That is, recess 24 is formed at the lowermost position in battery case 20. With such a configuration, moisture such as the dew condensation water inside battery case 20 flows toward recess 24 located at the lowermost position in battery case 20 when vehicle 200 travels. It should be noted that bottom surface 23a is a surface of bottom plate 23 on an inner side of battery case 20.

Bottom surface 24a extends orthogonal to the Z direction. Peripheral surface 24b includes a side surface 24c and a side surface 24d facing each other in the X direction. Side surface 24c is inclined so as to come closer to a side surface 24d side, from bottom surface 23a of bottom plate 23 toward bottom surface 24a of recess 24. Side surface 24d is inclined so as to come closer to a side surface 24c side, from bottom surface 23a toward bottom surface 24a. Although not shown, side surfaces of peripheral surface 24b facing each other in the Y direction are also formed in the same manner as side surface 24c and side surface 24d.

Absorber 60 is disposed in recess 24. Specifically, absorber 60 is in contact with each of bottom surface 24a and peripheral surface 24b that define recess 24. In detail, absorber 60 has a lower end surface 61, an upper end surface 62, and an outer peripheral surface 63. Lower end surface 61 is in contact with bottom surface 24a. Outer peripheral surface 63 is in contact with peripheral surface 24b. Outer peripheral surface 63 extends downward from a peripheral edge portion of upper end surface 62. Further, outer peripheral surface 63 extends upward from a peripheral edge portion of lower end surface 61. Outer peripheral surface 63 connects upper end surface 62 and lower end surface 61. It should be noted that lower end surface 61 and upper end surface 62 are examples of the "lower end portion" and the "upper end portion" in the present disclosure, respectively.

Absorber 60 is located below battery module 10. Specifically, upper end surface 62 of absorber 60 is located below bottom surface 23a of lower case 21 (bottom plate 23). That is, a space in which absorber 60 is not disposed is formed in the vicinity of an opening of recess 24. With such a configuration, the dew condensation water inside battery case 20 can easily flow into recess 24. It should be noted that the entire space in recess 24 may be filled with absorber 60.

Sensor unit 70 includes a printed substrate 71. Printed substrate 71 includes a substrate main body 71a, an electrode 72, and an electrode 73. Electrode 72 is connected to arithmetic unit 50 by wire 74. Electrode 73 is connected to arithmetic unit 50 by wire 75. It should be noted that electrode 72 and electrode 73 are not electrically connected by substrate main body 71a. Further, printed substrate 71 is an example of the "substrate" in the present disclosure.

Specifically, electrode 72 is electrically connected to a positive electrode of a power source 51 in arithmetic unit 50 by wire 74. Electrode 73 is electrically connected to a negative electrode of power source 51 in arithmetic unit 50 by wire 75. A current sensor 52 is disposed in a current path between power source 51 and wire 75. As power source 51, for example, a power source used to monitor the state of battery module 10 may be used.

Printed substrate 71 is disposed in recess 24. Specifically, printed substrate 71 is disposed inside absorber 60. That is, substrate main body 71a and electrodes 72 and 73 are completely shielded from outside air by absorber 60. Absorber 60 covers a void V between electrode 72 and electrode 73. In other words, void V is filled with absorber 60. Electrode 72 and electrode 73 are electrically connected by a portion of absorber 60 which fills void V. It should be noted that void V is an example of the "void between specific two points" in the present disclosure.

Printed substrate 71 (substrate main body 71a) measures a potential difference ΔV in the void between electrode 72 and electrode 73. Printed substrate 71 (substrate main body 71a) transmits information of measured potential difference ΔV to arithmetic unit 50. Printed substrate 71 (substrate main body 71a) communicates with arithmetic unit 50 in a wired manner (not shown) or a wireless manner.

Arithmetic unit 50 calculates a resistance value R in the void between electrode 72 and electrode 73, based on the information of potential difference ΔV received from printed substrate 71 and a detection value A of current sensor 52. Resistance value R is calculated using the following equation (1). $R = ΔV / A$ (1)

Resistance value R calculated by arithmetic unit 50 is based on a resistance value of the portion of absorber 60 provided in void V between electrode 72 and electrode 73. For example, resistance value R may be equal to the resistance value of the portion of absorber 60 provided in void V. Arithmetic unit 50 determines whether or not absorber 60 absorbs hydrogen sulfide, based on calculated resistance value R. Details will be described with reference to a flowchart described below.

Further, absorber 60 has a central portion 64 between upper end surface 62 and lower end surface 61. In Fig. 6, a broken line is drawn at a position P in the Z direction where central portion 64 is disposed. Sensor unit 70 (printed substrate 71) is disposed in a portion 65 of absorber 60 on a side closer to upper end surface 62 (the Z1 side) with respect to central portion 64.

Here, the hydrogen sulfide water has a specific gravity smaller than that of water. Therefore, when the hydrogen sulfide water and water are absorbed by absorber 60, the hydrogen sulfide water is likely to stay above the water in absorber 60. Therefore, by disposing sensor unit 70 in portion 65 of absorber 60 on the side closer to upper end surface 62 with respect to central portion 64, it is possible to easily detect a change in the electrical characteristic (resistance value) of absorber 60 caused by the hydrogen sulfide water.

### (Method for Detecting Hydrogen Sulfide)

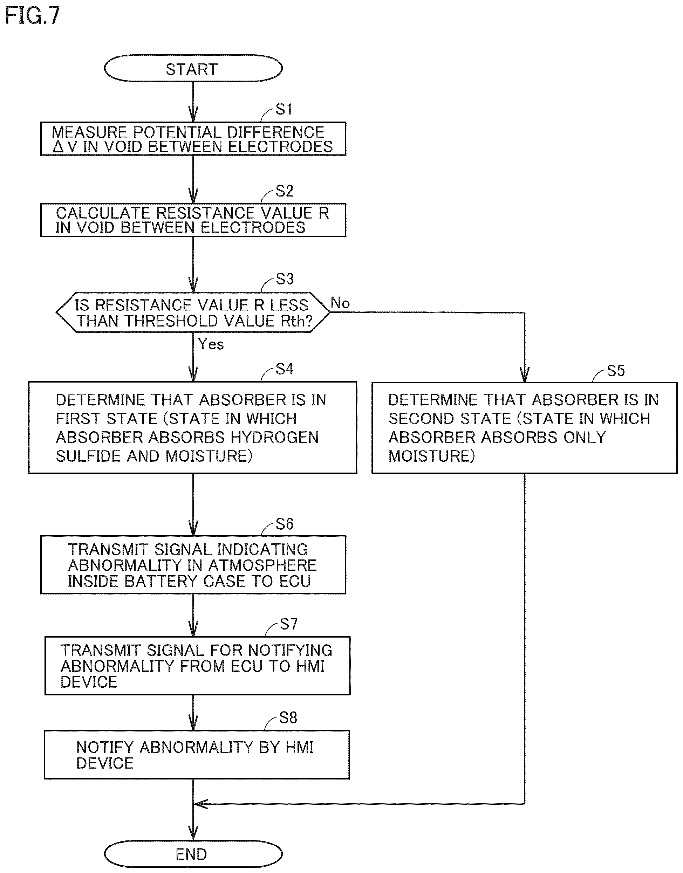

Fig. 7 is a flowchart showing a method for detecting hydrogen sulfide performed by battery pack 100. The control flow of Fig. 7 may be performed every predetermined period (for example, every five minutes). It should be noted that processing in steps S1 to S6 is performed by battery pack 100, and processing in step S7 and processing in step S8 are performed by ECU 150 and HMI device 120, respectively.

In step S1, potential difference ΔV in the void between electrode 72 and electrode 73 is measured by printed substrate 71 (substrate main body 71a).

In step S2, resistance value R in the void between electrode 72 and electrode 73 is calculated by arithmetic unit 50, using the information of potential difference ΔV measured in step S1 and the above equation (1).

In step S3, arithmetic unit 50 determines whether or not resistance value R calculated in step S2 is less than a predetermined threshold value Rth. When resistance value R is less than threshold value Rth (Yes in S3), the processing proceeds to step S4. When resistance value R is more than or equal to threshold value Rth (No in S3), the processing proceeds to step S5. It should be noted that threshold value Rth is an example of the "predetermined threshold value" in the present disclosure.

In step S4, arithmetic unit 50 determines that absorber 60 is in a first state. Here, threshold value Rth is a resistance value of absorber 60 when absorber 60 absorbs a certain amount or more of hydrogen sulfide and moisture, obtained beforehand through experiments, simulations, or the like. Therefore, the first state includes a state in which absorber 60 absorbs hydrogen sulfide and moisture. Next, the processing proceeds to step S6. It should be noted that threshold value Rth may be, for example, about 80% of an initial resistance value of absorber 60.

In step S5, arithmetic unit 50 determines that absorber 60 is in a second state. The second state includes a state in which absorber 60 absorbs moisture without absorbing hydrogen sulfide. Thereafter, the processing ends.

In step S6, arithmetic unit 50 transmits a signal indicating an abnormality in the atmosphere inside battery case 20 to ECU 150 (Fig. 1). Thereafter, the processing ends. It should be noted that, when ECU 150 receives the signal, ECU 150 transmits a signal for notifying the abnormality to HMI device 120 (Fig. 1).

Further, also when absorber 60 does not absorb hydrogen sulfide but absorbs a large amount of moisture, resistance value R may be lower than threshold value Rth. That is, the first state described above also includes a state in which absorber 60 does not absorb hydrogen sulfide but absorbs a large amount of moisture. Since the signal indicating an abnormality is also transmitted to ECU 150 in step S6 in this case, it is possible to notify a user of vehicle 200 of an abnormal state in which a large amount of moisture is present inside battery case 20. In other words, by using absorber 60, it is possible to sense not only generation of hydrogen sulfide but also generation of a large amount of dew condensation water. Therefore, the number of components can be reduced and the configuration of battery pack 100 can be simplified, when compared with a case where a sensor unit for detecting hydrogen sulfide and a sensor unit for detecting generation of a large amount of dew condensation water are separately provided.

In step S7, ECU 150 (Fig. 1) transmits the signal for notifying the user of the abnormality in the atmosphere inside battery case 20 to HMI device 120. In step S8, in response to receiving the signal in step S7, HMI device 120 may cause a car navigation device to display a message indicating the abnormality, or may notify the abnormality by voice through a speaker.

As described above, in the present embodiment, arithmetic unit 50 detects hydrogen sulfide absorbed by absorber 60, based on the resistance value of absorber 60 measured by sensor unit 70. Thereby, since it is easier and quicker to cause absorber 60 to absorb hydrogen sulfide, than to cause a wire or the like to be corroded by hydrogen sulfide, for example, it is possible to detect hydrogen sulfide more easily and quickly, when compared with a case where hydrogen sulfide is detected by detecting a voltage value of the wire or the like corroded by hydrogen sulfide.

Further, even when hydrogen sulfide is not detected by hydrogen sulfide sensor 40 because hydrogen sulfide is dissolved in the dew condensation water or because the amount of hydrogen sulfide generated from unit cell 11 (a generation amount per unit time) is small, hydrogen sulfide can be detected with a high sensitivity based on the resistance value of absorber 60.

Further, in the present embodiment, absorber 60 has water absorbency. Thereby, the hydrogen sulfide water formed by dissolving hydrogen sulfide in the dew condensation water inside battery case 20 can be absorbed by absorber 60. As a result, even when the concentration of hydrogen sulfide inside battery case 20 is low because hydrogen sulfide is absorbed by the dew condensation water inside battery case 20, generation of hydrogen sulfide can be easily determined based on the resistance value of absorber 60 that has absorbed the hydrogen sulfide water.

### (Modifications)

Although the above embodiment has described an example in which the resistance value of absorber 60 is calculated from the potential difference in the void between the two electrodes, and hydrogen sulfide is detected based on the resistance value, the present disclosure is not limited thereto. Hydrogen sulfide may be detected based only on the potential difference in the void between the two electrodes. For example, an acidity sensor (a pH sensor) may be used to measure a pH in the void between the electrodes from the potential difference in the void between the two electrodes, and hydrogen sulfide may be detected based on the measured pH.

Although the above embodiment has described an example in which printed substrate 71 and arithmetic unit 50 are connected in a wired manner, the present disclosure is not limited thereto. Printed substrate 71 and arithmetic unit 50 may communicate with each other in a wireless manner.

Although the above embodiment has described an example in which absorber 60 is disposed in recess 24, the present disclosure is not limited thereto. Absorber 60 may be disposed at a place other than recess 24 (for example, on bottom surface 23a of lower case 21).

Although the above embodiment has described an example in which absorber 60 is formed in a gel state (a solid state), the present disclosure is not limited thereto. For example, absorber 60 may be a liquid.

Although the above embodiment has described an example in which battery pack 100 includes hydrogen sulfide sensor 40 that detects hydrogen sulfide, the present disclosure is not limited thereto. Hydrogen sulfide sensor 40 does not have to be included in the battery pack.

Although the above embodiment has described an example in which entire printed substrate 71 is provided inside absorber 60, the present disclosure is not limited thereto. As long as electrode 72 and electrode 73 are electrically connected through absorber 60, portions of electrodes 72 and 73 and substrate main body 71a may be provided outside absorber 60. Further, printed substrate 71 (substrate main body 71a) does not have to be used. For example, only the two electrodes may be inserted into absorber 60.

Although the above embodiment has described an example in which sensor unit 70 (printed substrate 71) is disposed on the side closer to upper end surface 62 with respect to central portion 64 of absorber 60, the present disclosure is not limited thereto. Sensor unit 70 (printed substrate 71) may be disposed at central portion 64, or on a side closer to lower end surface 61 with respect to central portion 64.

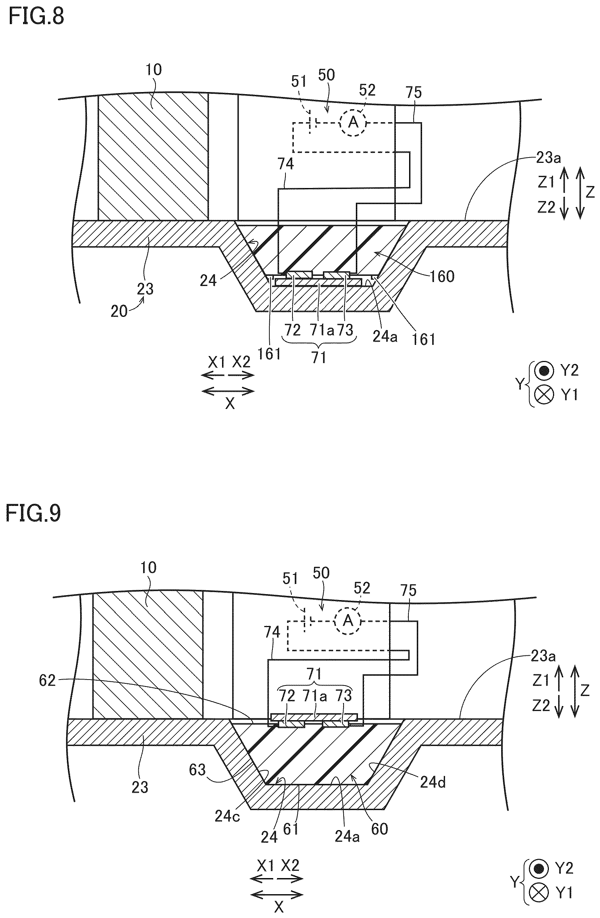

For example, as shown in Fig. 8, an absorber 160 may be spaced apart from bottom surface 24a of recess 24. Printed substrate 71 is disposed in a space between absorber 160 and bottom surface 24a. It should be noted that at least a portion of each of electrodes 72 and 73 is covered with absorber 160. Thereby, electrode 72 and electrode 73 are electrically connected by absorber 160. It should be noted that, in the example shown in Fig. 8, absorber 160 is supported from below by a support portion 161 provided to recess 24. Support portion 161 may be formed in an annular shape.

Further, as shown in Fig. 9, substrate main body 71a and portions of electrodes 72 and 73 may be provided outside from upper end surface 62 of absorber 60. As shown in Fig. 9, substrate main body 71a may be disposed above electrodes 72 and 73, and thereby entire substrate main body 71a may be provided outside absorber 60.

Although the above embodiment has described an example in which whether absorber 60 is in the first state or the second state is determined based on resistance value R of absorber 60, the present disclosure is not limited thereto. For example, it may also be possible to determine that absorber 60 is in a third state in which it does not absorb hydrogen sulfide water and moisture (a completely dried state). For example, determination that absorber 60 is in the third state may be made when resistance value R is less than or equal to a predetermined value smaller than threshold value Rth.

It should be noted that the modifications described above may be entirely or partially combined as appropriate for implementation.

Although the embodiment of the present disclosure has been described, it should be understood that the embodiment disclosed herein is illustrative and non-restrictive in every respect. The scope of the present disclosure is defined by the scope of the claims, and is intended to include any modifications within the scope and meaning equivalent to the scope of the claims.

## Claims

1. A power storage device (100) comprising:
a power storage module (10) including a power storage cell (11) having a sulfide solid electrolyte (3);
a case (20) that accommodates the power storage module;
an absorber (60, 160) that is disposed inside the case and absorbs sulfide gas;
a measurement unit (70) that measures an electrical characteristic of the absorber; and
a controller (50) that detects the sulfide gas absorbed by the absorber, based on the electrical characteristic measured by the measurement unit.

2. The power storage device according to claim 1, wherein the absorber has water absorbency.

3. The power storage device according to claim 1 or 2, wherein
the case includes a bottom plate (23) in which a recess (24) is formed, and
the absorber is disposed in the recess.

4. The power storage device according to claim 1 or 2, wherein the absorber is located below the power storage module.

5. The power storage device according to claim 1 or 2, wherein the absorber is formed in a gel state.

6. The power storage device according to claim 1 or 2, wherein the case has a sealed structure.

7. The power storage device according to claim 1 or 2, further comprising a gas detection unit (40) that detects the sulfide gas inside the case.

8. The power storage device according to claim 1 or 2, wherein
the absorber has an upper end portion (62), a lower end portion (61), and a central portion (64) between the upper end portion and the lower end portion, and
the measurement unit is disposed in a portion (65) of the absorber on a side closer to the upper end portion with respect to the central portion.

9. The power storage device according to claim 1 or 2, wherein
the measurement unit includes a substrate (71) disposed inside the case,
the absorber covers a void (V) between specific two points on the substrate,
the measurement unit measures a potential difference in the void between the two points covered with the absorber, and
the controller calculates a resistance value in the void between the two points based on the potential difference, and detects the sulfide gas absorbed by the absorber based on the resistance value.

10. The power storage device according to claim 9, wherein
when the resistance value is less than a predetermined threshold value, the controller determines that the absorber is in a first state, and
when the resistance value is more than or equal to the predetermined threshold value, the controller determines that the absorber is in a second state different from the first state.

11. The power storage device according to claim 10, wherein
the first state includes a state in which the absorber absorbs moisture and the sulfide gas, and
the second state includes a state in which the absorber absorbs moisture without absorbing the sulfide gas.

12. A method for detecting sulfide gas performed by a power storage device (100) including a power storage module (10) including a power storage cell (11) having a sulfide solid electrolyte (3), the method comprising:
measuring an electrical characteristic of an absorber (60, 160) that is disposed inside a case (20) accommodating the power storage module and absorbs the sulfide gas; and
detecting the sulfide gas absorbed by the absorber, based on the electrical characteristic.
